# EUROPEAN PATENT SPECIFICATION

(11) **EP 3 098 233 B1**
(45) Date of publication and mention of the grant of the patent: **06.11.2024**
(21) Application number: 14879409.2
(22) Date of filing: 27.01.2014
(51) Int. Cl.: C07K 14/505, C07K 1/20, A61K 38/19, A61P 7/00

(54) **RETICULOCYTE GROWTH FACTOR, AND PREPARATION METHOD AND APPLICATION THEREOF**
RETIKULOZYTENWACHSTUMSFAKTOR UND HERSTELLUNGSVERFAHREN UND ANWENDUNG DAVON
FACTEUR DE CROISSANCE DES RÉTICULOCYTES, PROCÉDÉ DE PRÉPARATION ET APPLICATION ASSOCIÉS

(30) Priority: 24.01.2014 CN 201410036118
(43) Date of publication of application: 30.11.2016
(73) Proprietor: Nanjing Biyoukang Biotechnology Co. Ltd., Nanjing, Jiangsu 211100 (CN)
(72) Inventor: JIN, Yifeng, Nanjing Jiangsu 211100 (CN); WU, Qi, Nanjing Jiangsu 211100 (CN); ZHANG, Taiping, Nanjing Jiangsu 211100 (CN); MA, Xiaoyan, Nanjing Jiangsu 211100 (CN); XUAN, Ji, Nanjing Jiangsu 211100 (CN)
(74) Representative: Engelhard, Markus
(86) International application number: PCT/CN2014/071586
(87) International publication number: WO 2015/109604

(56) References cited:
- WO-A1-2010/085879
- WO-A2-2013/025538
- WO-A2-2013/025538
- I GRAZIADEI ET AL: "The acute-phase protein alpha 1-antitrypsin inhibits growth and proliferation of human early erythroid progenitor cells (burst-forming units-erythroid) and of human erythroleukemic cells (K562) in vitro by interfering with transferrin iron uptake", BLOOD, 1 January 1994 (1994-01-01), UNITED STATES, pages 260, XP055369143, Retrieved from the Internet <URL:http://www.bloodjournal.org/content/83/1/260.full.pdf>
- QIN, H. ET AL.: "Highly Efficient Extraction of Serum Peptides by Ordered Meso- porous Carbon", ANGEWANDTE CHEMIE INT. EDITION, vol. 50, no. 51, 16 December 2011 (2011-12-16), pages 12218 - 12221, XP055424842, ISSN: 1521-3773, DOI: 10.1002/anie.201103666
- GRAZIADEI, I: "The Acute-Phase Protein a 1-Antitrypsin Inhibits Growth and Proliferation of Human Early Erythroid Progenitor Cells (Burst-Forming Units-Erythroid) and of Human Erythroleukemic Cell (sK 562) In Vitro by Interfering With Transferrin Iron Uptake", BLOOD, vol. 83, no. 1, 1 January 1994 (1994-01-01), pages 260 - 268, XP055369143, ISSN: 1528-0020

## Description

### Technical Field

The present invention relates to the field of medical biotechnology, and in particular relates to a reticulocyte growth factor and a preparing method and application thereof.

### Related Art

At present, the most efficient medicine for clinically treating severe anemia is Erythropoietin (rhu-EPO). Since the recombinant human (rhu) EPO of the Amgen Company in America was approved by FDA in 1989, the rhu-EPO has been generally commonly accepted, and has developed to the most successful example in DNA recombinant medicine. EPO is one unique special red cell increasing factor in generation of red cells as so far. Although it is reported that Stem Cell Growth Factor (SCF) and Colony Stimulating Factors (CSF_{S}) also have a promoting action for the red cells, their functions are diversified and not exclusive. At present, no new medicine for treating the severe anemia is found yet.

### SUMMARY

The present invention aims to provide a new cell growth factor - Reticulocyte Growth Factor (RGF), and a preparing method and application thereof.

In a research process of the present invention, in kidney cell culture fluid, proteins with erythropoiesis activity exist in both human urine and plasma, but their molecular weights, immunogenicity and even receptors are different from EPO. The content of such active proteins is very low, it is difficult to obtain enough pure products for quality research, and finally, it is found that impurities from an α₁-antitrypsin preparation of the human plasma has high activity for promoting generation of reticulocyte. Several high-activity proteins, obtained by biochemical and purifying means such as High Performance Liquid Chromatography (HPLC), are peptide fragments of different lengths in a precursor of the α₁-antitrypsin, and have carbohydrates of different lengths, and some components have few or no carbohydrate.

### Technical solutions of the present invention

### 1. Purification of RGF

a, the α₁-antitrypsin preparation (A-9024) of Sigma Company is from human plasma, and has very high activity for promoting generation of the reticulocyte. Activity inspection adopts a mouse reticulocyte determining method of professor CHENG Yaqin, which is described as follows:
animals: ICR mice, weight: 18-22kg, male
samples: diluting according to the activity of RGF, 5-20µg/ml in common
method: dividing three mice in one group, intramuscularly injecting 0.06ml, 0.12ml and 0.24ml, injecting for three days continuously, sampling eye socket blood on the fourth day, adding 7-8 drops into a tube with a lid filled with 20µl 100U/ml of heparin sodium, mixing uniformly, taking out 50µl blood, dropwise adding on dried brilliant cresyl blue dye precoated at one end of a glass slide, fully mixing, and placing in a 37°C wet box for 30 minutes; pushing single layer of cells on a second glass slide, after naturally drying, fixing with methyl alcohol, then redyeing through Wright's stain, dropwise adding 0.02mol/LPBS (PH6.8), avoiding drying up, and washing the dye liquor with PBS or distilled water after 30 minutes;
reading a film by a high power oil immersion lens, where a nucleus substance in the reticulocyte can be dyed to be deep blue color dot-like, line-like, chain-like or cluster-like, while the red cells do not contain such substance; counting 500-1000 red cells in common and calculating the percentage of the reticulocyte; reading two films for each sample, averaging, then averaging again for three mice in each group, and subtracting the percentage of the reticulocyte in a control group (0.12µl normal saline) to obtain the net increase percentage of the reticulocyte, defining every 1% of increase as one unit (U), and calculating specific activity by injected protein content (once), namely taking the rising unit stimulated by each mg of protein (U/mg) as the specific activity;
preparing the α₁-antitrypsin preparation into different concentrations with normal saline, taking 15 ICR male mice (weight: 18-22g), dividing into 5 groups each of which contains 3 mice, intramuscularly injecting 0.12ml normal saline into the control group, injecting 0.12ml antitrypsin preparation of different concentrations into the rest growped mice, injecting for three days continuously, sampling eye socket blood on the fourth day, dyeing by brilliant cresyl blue, and calculating the percentage of the reticulocyte in the red cells with the oil immersion lens, wherein a result shows that the activity for promoting the generation of the reticulocyte in the antitrypsin preparation is very high, and can serve as an initial material for further separation and purifying. The result is as shown in Table 1:

**Table 1 Activity for promoting generation of reticulocyte in α₁-antitrypsin preparation**

| α₁-antitrypsin preparation concentration | Reticulocyte% | Net increase reticulocyte% |
|---|---|---|
| 0 | 1.8 | |
| 2µg/ml | 8.2 | 6.4 |
| 5µg/ml | 6.6 | 4.6 |
| 20µg/ml | 16.45 | 14.65 |
| 80µg/ml | 1.8 | 0 |

| | | |
|---|---|---|
| The data indicates that the α₁-antitrypsin preparation indeed contains higher activity for promoting generation of reticulocyte. | | |

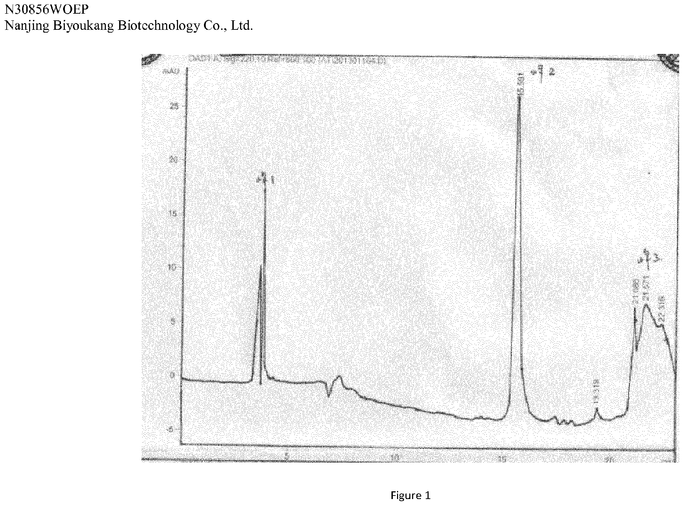

b, dissolving the α₁-antitrypsin into 2mg/ml with PBS of 0.02mol/L, performing neutral separating by an HPLC C₁₈ reversed-phase column to obtain 3 peak groups, wherein, through determining, only the 3rd peak group has activity, a C18 reversed-phase column separation pattern is as shown in FIG. 1 and a result is as shown in Table 2:

**Table 2 Activities of respective parts obtained by separating the α₁-antitrypsin through the HPLC C₁₈ reversed-phase column**

| Collected parts | Reticulocyte % | Net increase reticulocyte% |
|---|---|---|
| Control saline 0.12ml | 2.4 | |
| Peak 1 20µg/ml 0.12ml | 3.3 | 0.9 |
| Peak 2 20µg/ml 0.12ml | 4.48 | 2.08 |
| Peak 3 20µg/ml 0.12ml | 8.6 | 6.2 |

c, increasing an eluting gradient of the peak 3 group with the C18 reversed-phase column again to obtain 10 collected parts, wherein an activity inspection result shows that the 8th collected part has the highest activity, the 7th and 9th collected parts have the activity second to that of the 8th collected part, and the other 7 collected parts have no activity. A separating pattern is as shown in FIG. 1, and an activity determining results are shown in Table 3:

**Table 3 Activities of 7^{#}, 8^{#}, 9^{#} and 10^{#} parts in the 10 collected parts obtained by separating the third peak for the second time**

| Collected parts | Reticulocyte % | Net increase reticulocyte% |
|---|---|---|
| Control saline 0.12ml | 1.2 | |
| Peak 7^{#} 20µg/ml 0.12ml | 4.9 | 3.7 |
| Peak 8^{#} 20µg/ml 0.12ml | 8.3 | 7.1 |
| Peak 9^{#} 20µg/ml 0.12ml | 5.8 | 4.6 |
| Peak 10^{#} 20µg/ml 0.12ml | 4.1 | 2.9 |

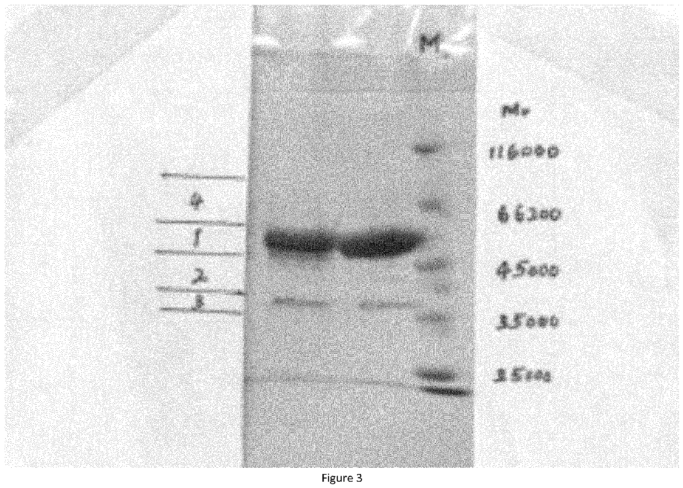

d, performing preparative SDS-PAGE as shown in FIG. 3) on the eighth collected part, cutting a piece of gel, according to the position displayed by staining, for the undyed gel, cut first to fourth bands of gel, respectively triturating, extracting with water for 24 hours, extracting for three times continuously, combining supernate, determining activity after freeze-drying, wherein, only the bands 1, 2 and 3 extracting liquor show activity, the band 4 has no activity, a result is as follows:

**Table 4 Activities of extracting liquors of respective bands cut by electrophoresis and prepared by the activity peak eighth sample after HPLC separation**

| Prepared electrophoresis bands | Reticulocyte % | Net increase reticulocyte% |
|---|---|---|
| Control saline 0.12ml | 1.2 | |
| Band 1 20µg/ml 0.12ml | 10.7 | 9.2 |
| Band 2 20µg/ml 0.12ml | 8.1 | 6.6 |
| Band 3 10µg/ml 0.12ml | 7.2 | 5.7 |
| Band 4 20µg/ml 0.12ml | 1.7 | 0.2 |

then performing SDS-PAGE inspection after freeze-drying of the plurality of extracting liquors, wherein the bands 1 and 3 have clear dyed strips, the band 2 is high in activity but is close to the band 1, possibly has pollution, is fuzzily dyed, and is obvious not single bands, therefore, samples of the bands 1 and 3 are sent to the Suzhou ProtTech Inc. for sequencing, an identification result shows that the bands 1 and 3 are a precursor of the α₁-antitrypsin, we called the peptide fragment in such family as Reticulocyte Growth Factors (RGF family), and the sequence of the precursor is:

Met Pro Ser Ser Val Ser Trp Gly Ile Leu Leu Leu Ala Gly Leu
1               5                   10                  15
Cys Cys Leu Val Pro Val Ser Leu Ala Glu Asp Pro Gln Gly Asp
                20                  25                  30
Ala Ala Gln Lys Thr Asp Thr Ser His His Asp Gln Asp His Pro
                35                  40                  45
Thr Phe Asn Lys Ile Thr Pro Asn Leu Ala Glu Phe Ala Phe Ser
                50                  55                  60
Leu Tyr Arg Gln Leu Ala His Gln Ser Asn Ser Thr Asn Ile Phe
                65                  70                  75
Phe Ser Pro Val Ser Ile Ala Thr Ala Phe Ala Met Leu Ser Leu
                80                  85                  90
Gly Thr Lys Ala Asp Thr His Asp Glu Ile Leu Glu Gly Leu Asn
                95                  100                 105
Phe Asn Leu Thr Glu Ile Pro Glu Ala Gln Ile His Glu Gly Phe
                110                 115                 120
Gln Glu Leu Leu Arg Thr Leu Asn Gln Pro Asp Ser Gln Leu Gln

                125                 130                 135
Leu Thr Thr Gly Asn Gly Leu Phe Leu Ser Glu Gly Leu Lys Leu
135 140 145
Val Asp Lys Phe Leu Glu Asp Val Lys Lys Leu Tyr His Ser Glu
150 155 160
Ala Phe Thr Val Asn Phe Gly Asp Thr Glu Glu Ala Lys Lys Gln
165 170 175
Ile Asn Asp Tyr Val Glu Lys Gly Thr Gln Gly Lys Ile Val Asp
180 185 190
Leu Val Lys Glu Leu Asp Arg Asp Thr Val Phe Ala Leu Val Asn
195 200 205
Tyr Ile Phe Phe Lys Gly Lys Trp Glu Arg Pro Phe Glu Val Lys
210 215 220
Asp Thr Glu Glu Glu Asp Phe His Val Asp Gln Val Thr Thr Val
225 230 235
Lys Val Pro Met Met Lys Arg Leu Gly Met Phe Asn Ile Gln His
240 245 250
Cys Lys Lys Leu Ser Ser Trp Val Leu Leu Met Lys Tyr Leu Gly
255 260 265
Asn Ala Thr Ala Ile Phe Phe Leu Pro Asp Glu Gly Lys Leu Gln
270 275 280
His Leu Glu Asn Glu Leu Thr His Asp Ile Ile Thr Lys Phe Leu
285 290 295
Glu Asn Glu Asp Arg Arg Ser Ala Ser Leu His Leu Pro Lys Leu
300 305 310
Ser Ile Thr Gly Thr Tyr Asp Leu Lys Ser Val Leu Gly Gln Leu
315 320 325
Gly Ile Thr Lys Val Phe Ser Asn Gly Ala Asp Leu Ser Gly Val
330 335 340
Thr Glu Glu Ala Pro Leu Lys Leu Ser Lys Ala Val His Lys Ala

345 350 355
Val Leu Thr Ile Asp Glu Lys Gly Thr Glu Ala Ala Gly Ala Met
360 365 370
Phe Leu Glu Ala Ile Pro Met Ser Ile Pro Pro Glu Val Lys Phe
375 380 385
Asn Lys Pro Phe Val Phe Leu Met Ile Glu Gln Asp Thr Lys Ser
390 395 400
Pro Leu Phe Met Gly Lys Val Val Asn Pro Thr Gln Lys
                410                 415

### 2. Chemical properties of RGF

In order to determining the chemical properties of the bands 1 and 3 prepared by SDS-PAGE, the gel bands displayed by staining were sent to the Suzhou ProtTech Inc. for determining the sequences of the N end and C end, a result shows that the band 1 (RTF-6 in a sequencing report of the Suzhou ProtTech Inc.) contains four components which are fragments of different lengths of the precursor of the α₁-antitrypsin, N end sites have slight difference, the C ends are same as the C ends of precursor, band 3 (RTF-8 in a report of the Suzhou ProtTech Inc.) contains only one component, the N end and C end are totally same as the bands 1-2 and contain 394 amino acids in total from the 25 site to the 418 site, the band 1 has a molecular weight of about 50000, which is 10000 larger than the band 3, it is known that the band 1 contains carbohydrate, the components in the band 1 are glycoproteins, the band 3 does not contain carbohydrate through speculation from the molecular weight, or contains extremely less carbohydrate.

The RGF family is a group of protein fragments with different lengths and different carbohydrate contents from the precursor of the α₁-antitrypsin. An amino acid sequence thereof is:
N end (band1-1) 
24
Ala Glu Asp Pro Gln Gly Asp Ala Ala Gln Lys Thr Asp Thr
Ser His His Asp Gln Asp His Pro Thr Phe Asn Lys
49
N end (band 1-2) 
25
Glu Asp Pro Gln Gly Asp Ala Ala Gln Lys Thr Asp Thr Ser His His
Asp Gln Asp His Pro Thr Phe Asn Lys
49
 N end (1-3) 
27
Pro Gln Gly Asp Ala Ala Gln Lys Thr Asp Thr Ser His His
Asp Gln Asp His Pro Thr Phe Asn Lys
49
N end (band 1-4) 
30
Asp Ala Ala Gln Lys Thr Asp Thr Ser His His Asp Gln Asp His Pro
Thr Phe Asn Lys
49
 C fragment

412
Val Val Asn Pro Thr Gln Lys
418

### 3. Biological properties of RGF

a, therapeutic action of the RGF for aplastic anemia mice, this experiment is entrusted to the Pharmacology Teaching and Research Department of the Institute of Traditional Chinese medicine, Jiangsu,
modeling: Injecting 100mg/kg of cyclophosphamide ip into male mice of 18-22g for three continuous days
test: the mice are divided into 5 groups and EPO is domestic (NING Hongxin). The RGF is the produc prepaed in this laboratory, and a result of RBC and WBC examining after continuous injecting for 8 days is as shown in Table 5:

**Table 5 Therapeutic action of the RGF to aplastic anemia mice**

| Groups | Mouse sample number | Dosage | Result | | Red cell promotion % | Leukocyte promotion % |
|---|---|---|---|---|---|---|
| | | | RBC | WBC | | |
| Blank control | 20 | Normal saline | 487± | 190± | | |
| | | | 149.3 | 71.8 | | |
| Aplastic anemiamodel group | 20 | Normal saline | 305±102.5 | 115±36.5 | | |
| EPO group in model group | 20 | 333U/kg | 429±94.8 | 201±79.6 | 40.66 | 77.78 |
| RGF group in model group | 20 | 167µg/kg | 442±105.1 | 195±81.1 | 40.92 | 69.57 |
| EPO group +RGF group in model group | 20 | 1/2EPO±1/2RPF | 516±108.4 | 233±104.5 | 69.18 | 102.61 |

| | | | | | | |
|---|---|---|---|---|---|---|
| The cyclophosphamide can obviously cause anemia to the mice, gneration of the red cells can be promoted by injecting the EPO or RGF, for example, the therapeutic action, achieved by half amount of EPO and half amount of RGF, for co-treating the generation of the red cells is better than the therapeutic action achieved by single whole amount of the RGF or EPO, an obvious synergistic effect is achieved, and it should be noted that the RGF or EPO further has a promotion action for the generation of leukocytes, and have the same synergistic effect. | | | | | | |

b, synergistic effect of the RGF and EPO for generation of mouse reticulocyte animals: 30 ICR mice, male, weight: 18-22g, 10 groups, 3 mice in each group
experiment: preparing the RGF and EPO into three following concentrations: (a) RGF5µg/ml; (b) EPO (rhuEPO of Amgen Company) 18U/ml; (c) balanced mixing of the RGF and EPO; injecting the RGF and EPO with the concentrations of the a, b and c into the mice in each group by 0.06ml, 0.12ml and 0.24ml for continuous 3 days, sampling eye socket blood on the fourth day, smearing, dyeing, and examining the increase percentage of the reticulocyte with a microscope, wherein a result is as shown in Table 6:

**Table 6 Synergistic effect of RGF and EPO (Amgon Company)**

| Injecting dosage (each mouse every day) | Net increase reticulocyte% | | |
|---|---|---|---|
| | EPO (18U/ml) | RGF (5µg/ml) | EPO9U/ml RGF2.5fµg/ml |
| 0.06ml | 4.8% | 4.9% | 5.3% |
| 0.12ml | 6.2% | 6.6% | 7.2% |
| 0.24ml | 7.4% | 7.8% | 11.3% |

| | | | |
|---|---|---|---|
| The RGF and EPO both have well activity for promoting the generation of the reticulocyte, 18U/ml of EPO and 5µg/ml of RGF are very close in activity, after the EPO and the RGF are mixed in the same amount, an obvious synergistic effect is generated, especially, the test group injected with the 0.24ml is higher than the test group injected with the 0.24ml of the EPO or RGF by about 50% in reticulocyte increase, then is this phenomenon possibly the false appearance caused by the generation of the mouse EPO caused by the RGF in vivo, for this purpose, we design a process that after the RGF of different dosages is injected to the mice for 3 days, the EPO level in blood of the mice is determined by monoclonal antibody to compare with the normal saline control group, the EPO level is not increased, so that such possibility is excluded. It is inferred that the EPO and the RGF act in different development stages of the red cells, under a relay action of the EPO and the RGF, the speed of the red cells is naturally increased. This hypothesis is verified in follow fluorescence labeling experiment. | | | |

The synergistic effect of the RGF and EPO occurs in the two experiments, and this phenomenon has great significance in treatment of the severe anemia and indicates a potential of more reasonably and more effectively treating the severe anemia.
c, fluorescence labeling experiment
labeling the EPO with fluorochrome rhodamine (RB200), wherein the EPO is red under the observation of a fluorescence microscope, labeling the RGF with fluorochrome FITC, wherein the RGF is green under the observation of the fluorescence microscope, sampling about 2ml of bone marrow cells from longs bones of four mice, centrifuging at 1000r/m for four minutes, pouring off supernatant, resuspending in 6ml of Hank'S liquid, separating through a lymphocyte separating medium, and taking and centrifuging interface cells; diluting the cells with RPMI1640 into 1.8×10⁶/ml of cell suspension, taking 5ml of cell suspension, centrifuging, resuspending in 0.3ml of PBS, adding 60µl of FITC-RGF and 60µl of RB200-EPO, placing in a refrigerator for 30 minutes, wherein, by observing under the fluorescence microscope, most cells are doubly labeled, a few cells are singly labeled by FITC-RGF, but there are no cells labeled by RB200-EPO. This phenomenon prompts that the RGF is used in an earlier stage of a cell development process, an EPO receptor does not occur till the cells are developed to the next stage under the promotion of the RGF, therefore, the dual-receptor cells on which the RGF and EPO show at the cells occur.

### Beneficial effects of the present invention

Differentiation and maturation of the red cells are a multi-stage multifactor-participated complicated process, however, a second factor for promoting the generation of the red cells is not found for last 20 years, so that the finding of the reticulocyte growth factor has great significance. When the reticulocyte growth factor and EPO are combined in use, the effect is better than that when the reticulocyte growth factor or EPO is singly used. It is indicated that different development stages of the red cells need different induction and promotion of differnet factors, which is a new start for us to learn the total development process of the red cells, and treat the severe anemia. The present invention helps to explain the synergistic effect of the RGF and EPO occurring in normal mice and aplastic anemia mice. Because they act in different development stages of the red cells, and bone marrow cells generate more EPO receptors under the action of the RGF, and thus are developed to dual-receptor cells, the EPO then continuous acts, and the better effect can be understood under a relay action. The invnetion develops a new medicine for treating the severe anemia.

### BRIEF DESCRIPTION OF THE DRAWINGS

FIG. 1 is a chromatography pattern of an α₁-antitrypsin separated by an HPLC C₁₈ reversed-phase column in first separation, wherein 3 collected parts are obtained from the first separation, and the 3rd part has activity.
FIG. 2 is 10 collected parts obtained by the HPLC C₁₈ reversed-phase column in second separation larger than the first separation by gradient, wherein the eighth peak collected part pointed by the arrow has higher activity.
FIG. 3 is an SDS-PAGE diagram of an eighth part in the ten collected parts in the second HPLC separation, wherein a molecular weight is about 50000, the part dyed with the deepest color is band 1 (RGF-B1), the molecular weights of about 40000 are called as band 3 (RGF-B3), the part between the B1 and B3 is called as RGF-B2, and the parts above B1 have no activity.

### DETAILED DESCRIPTION

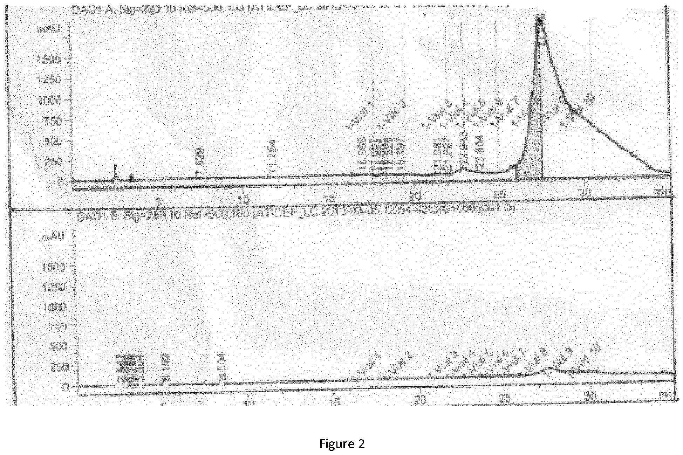

1, taking two bottles of α₁-antitrypsin, dissolving in pH7.0 0.02MOL/L of PBS with the concentration of 2.5mg/ml, performing neutral separation by an High Performance Liquid Chromatography (HPLC) C18 column, and obtaining 3 peak groups, wherein, as shown in FIG. 1, the 3rd peak group shows a strong action for promoting the generation of reticulocyte;
2, re-separating the third peak group, but pulling the separated eluting gradient, and obtaining 10 collected parts, wherein only the 7th, 8th and 9th collected parts show the activity for promoting the generation of the reticulocyte, the 8th collected part has the highest activity as shown in FIG. 2 and FIG. 3; performing preparative SDS-PAGE on the 8th collected part, longitudinally cutting a piece of gel, dyeing by Coomassie Brilliant Blue to determine the position of proteins, wherein a deeper and wider band occurs at the position of the molecular weight of about 50000; cutting the corresponding gel block as band 1, cutting a clear band at the position of the molecular weight of about 40000 as band 3, wherein, 3-4 dyed bands between the band 1 and band 3 are fuzzy, one of the bands is close to number 1, and is called as band 2, and the band between the molecular weights of 50000-68000 is called as band 4; crushing the gel of the four parts with glass rods individually, extracting for 24 hours with water, sucking out supernatant, and repeating for three times; combining and freeze-drying the extracting liquors obtained at four times on the 5th day, and dissolving with a small amount of water and then determining the activity, wherein a result shows that the extracting liquors of the bands 1, 2 and 3 have high activity, and the band 4 has no activity, and the result is as shown in table 3;
4, performing SDS-PAGE on the extracting liquors of the bands 1, 2 and 3, wherein the bands 1 and 3 have clear strips after staining, the band 2 has fuzzy strips, some strips are very close to the band 1 in position, therefore, the dyeing gel of the bands 1 and 3 are sent to the ProtTech Inc. for sequencing, a determining result shows that the bands 1 and 3 are parts of a precursor of the α₁-antitrypsin and a result is as shown in the amino acid sequence;
5, in order to determine the chemical properties of the bands 1 and 3, the SDS-PAGE, drying gel blocks of the band 1 and band 3 are sent to the Suzhou ProtTech Inc. for sequencing, and a result is as shown in attachment 2. The N end and C end sequencing result shows that four components contained in the band 1 are fragments of different lengths in the precursor of the α₁-Antitrypsinm, the precursor is composed of 418 amino acids, these fragments have different start points of the N ends, and are respectively 24, 25, 27 and 30 site amino acids; the C ends are same as the tail ends of the precursor. The band 3 contains single component, is totally same as the band 1-2 in structure and is composed of 394 amino acids in total from the 25h site to the 418th site. But the molecular weight is smaller than band 1 that contains carbohydrate, and based on the molecular weight, the band 3 contains no or extremely less carbohydrate;
6, the sequence of the precursor of the α₁-Antitrypsin is known, the molecular weight of antitrypsin is known as 55000 - 56000, the bands 1 and 3 are both smaller than the precursor in molecular weight, and plural components between the molecular weights of 50000-40000 also show activity. It is estimated that the precursor of the Antitrypsin can be degraded into a group of peptide fragments in different lengths, and these peptide fragments also have carbohydrate chains of different lengths or no carbohydrate. It is still unknown yet that whether the active protein groups composed of the peptide fragments have different action sites, but all the components of the antitrypsin family participate in the action of regulating the generation of the red cells in organism together with the EPO.

## Claims

1. A composition comprising a peptide fragment from the precursor of α₁-antitrypsin, wherein said fragment is a reticulocyte growth factor and consists of an amino acid sequence of 394 amino acids of SEQ ID NO. 1;
and wherein said fragment has an N terminus of 
Glu Asp Pro Gln Gly Asp Ala Ala Gln Lys Thr Asp Thr Ser His His
Asp Gln Asp His Pro Thr Phe Asn Lys
and a C terminus of Val Val Asn Pro Thr Gln Lys,
and does not contain carbohydrate.

2. A preparing method of the composition according to claim 1, comprising the following steps:
(1) dissolving α₁-antitrypsin preparation from human plasma into 2.5mg/ml with PBS of 0.02mol/L;
(2) obtaining 3 protein peak groups through a separation by an HPLC C_{18F} reverse-phase column, wherein through activity determination, the third peak group has an activity of promoting the growth of reticulocyte;
(3) separating the third peak group through a second HPLC C_{18F} process, wherein 10 protein collected parts are obtained when an eluting gradient is increased, and the 8th part was proved having higher activity through activity determination;
(4) performing preparative SDS-PAGE on the 8th collected part, cutting gel with the obvious protein and crushing, repeatedly extracting with water, detecting purity and activity after concentration, and sequencing.

3. The composition according to claim 1 for use in the treatment of anemia.

4. The composition according to claim 1 in combination with erythropoietin for use in the treatment of anemia.

## Patentansprüche

1. Zusammensetzung, umfassend ein Peptidfragment des Vorläufers von α₁-Antitrypsin, wobei das Fragment ein Retikulozyten-Wachstumsfaktor ist und aus einer Aminosäuresequenz von 394 Aminosäuren der SEQ ID NO. 1 besteht;
und wobei das Fragment einen N-Terminus hat von 
Glu Asp Pro Gln Gly Asp Ala Ala Gln Lys Thr Asp Thr Ser His His
Asp Gln Asp His Pro Thr Phe Asn Lys
und einen C-Terminus von Val va1 Asn Pro Thr Gin Lys,
und kein Kohlenhydrat enthält.

2. Herstellungsverfahren für die Zusammensetzung nach Anspruch 1, umfassend die folgenden Schritte:
(1) Auflösen eines α₁-Antitrypsin-Präparats aus menschlichem Plasma in 2,5 mg/ml mit PBS von 0,02 mol/L;
(2) Erhalten von 3 Proteinpeak-Gruppen durch eine Auftrennung mittels einer HPLC-C_{18F}-Umkehrphasen-Säule, wobei durch Aktivitätsbestimmung die dritte Peak-Gruppe eine Aktivität zur Förderung des Wachstums von Retikulozyten hat;
(3) Auftrennen der dritten Peak-Gruppe durch ein zweites HPLC-C_{18F}-Verfahren, wobei 10 gesammelte Proteinanteile erhalten werden, wenn ein Elutionsgradient erhöht wird, und der achte Anteil nachweislich eine höhere Aktivität gemäß Aktivitätsbestimmung aufweist;
(4) Durchführung einer präparativen SDS-PAGE mit dem achten gesammelten Anteil, Schneiden des Gels mit dem erkennbaren Protein und Zerkleinern, wiederholtes Extrahieren mit Wasser, Nachweisen der Reinheit und Aktivität nach der Konzentrierung, und Sequenzierung.

3. Zusammensetzung nach Anspruch 1 zur Verwendung bei der Behandlung von Anämie.

4. Zusammensetzung nach Anspruch 1 in Kombination mit Erythropoietin zur Verwendung bei der Behandlung von Anämie.

## Revendications

1. Composition comprenant un fragment peptidique du précurseur de l'α₁-antitrypsine, dans laquelle ledit fragment est un facteur de croissance des réticulocytes et consiste en une séquence d'acides aminés de 394 acides aminés de SEQ ID NO: 1 ;
et dans laquelle ledit fragment possède une extrémité N-terminale de 
Glu Asp Pro Gln Gly Asp Ala Ala Gln Lys Thr Asp Thr Ser His His
Asp Gln Asp His Pro Thr Phe Asn Lys
et une extrémité C-terminale de Val Val Asn Pro Thr Gln Lys,
et ne contient pas de glucide.

2. Procédé de préparation de la composition selon la revendication 1, comprenant les étapes suivantes :
(1) la dissolution d'une préparation d'α₁-antitrypsine à partir de plasma humain dans 2,5 mg/ml avec du PBS à 0,02 mol/l ;
(2) l'obtention de 3 groupes de pics de protéines via une séparation par une colonne HPLC en phase inverse C_{18F}, dans lequel, via une détermination de l'activité, le troisième groupe de pics possède une activité favorisant la croissance des réticulocytes ;
(3) la séparation du troisième groupe de pics via un deuxième processus de HPLC C_{18F}, dans lequel 10 parties collectées de protéines sont obtenues lorsqu'un gradient d'élution est augmenté, et la 8^{e} partie s'avère posséder une activité plus élevée via une détermination de l'activité ;
(4) la réalisation d'un SDS-PAGE préparatif sur la 8^{e} partie collectée, une coupe du gel comportant la protéine évidente et un broyage, une extraction répétée avec de l'eau, une détection de la pureté et de l'activité après concentration, et un séquençage.

3. Composition selon la revendication 1 destinée à être utilisée dans le traitement de l'anémie.

4. Composition selon la revendication 1, en combinaison avec de l'érythropoïétine pour une utilisation dans le traitement de l'anémie.
